# EUROPÄISCHE PATENTSCHRIFT

(11) **EP 0 448 681 B1**
(45) Veröffentlichungstag und Bekanntmachung des Hinweises auf die Patenterteilung: **26.02.1997**
(21) Anmeldenummer: 90915426.2
(22) Anmeldetag: 17.10.1990
(51) Int. Cl.: G01N 27/12

(54) **GAS-SENSOR-ANORDNUNG**
GAS SENSOR ARRANGEMENT
DISPOSITIF DETECTEUR DE GAZ

(30) Priorität: 17.10.1989 DE 3934532; 23.06.1990 DE 4020113; 08.08.1990 DE 4025117; 31.08.1990 DE 4027547
(43) Veröffentlichungstag der Anmeldung: 02.10.1991
(60) Teilanmeldung: 96112470.8
(73) Patentinhaber: I.T.V.I. INTERNATIONAL TECHNO VENTURE INVEST AG, 9490 Vaduz (LI)
(72) Erfinder: RUMP, Hanns, D-4750 Unna-Massen (DE); KOHL, Claus-Dieter, D-5120 Herzogenrath 3 (DE)
(74) Vertreter: Spalthoff, Adolf, Dipl.-Ing.
(86) Internationale Anmeldenummer: EP9001752
(87) Internationale Veröffentlichungsnummer: WO9106001

(56) Entgegenhaltungen:
- DE-A- 3 836 819
- DE-C- 3 213 286
- US-A- 4 169 369
- US-A- 4 792 433
- Patent Abstracts of Japan, Band 10, Nr 93, P445, Zusammenfassung von JP 60-227160, publ 1985-11-12 (SHIN COSMOS DENKI K.K.)

## Beschreibung

Die Erfindung bezieht sich auf eine Gas-Sensor-Anordnung nach dem Oberbegriff des Patentanspruchs 1.

Die Notwendigkeit, Gase in der Luft nachzuweisen, wird immer wichtiger.

Es besteht z.B. der Wunsch, in Abhängigkeit vom Schadstoffgehalt der Luft Schaltvorgänge auszulösen. Insbesondere bei der Belüftung von Kraftfahrzeugen sind zahlreiche Bemühungen bekannt, mit Hilfe eines gasempfindlichen Sensors so auf die Lüftung des Kraftfahrzeugs einzuwirken, daß bei Bestehen einer ungewöhnlich hohen Schadstoffbelastung der Außenluft die Frischluftzufuhr des Kraftfahrzeugs unterbrochen und z.B. auf Umluftbetrieb umgeschaltet wird.

Es ist bekannt, mit Hilfe von Metalloxid-Gas-Sensorelementen bestimmte Gase zu detektieren. Dabei besteht das Sensorelement meist aus einem Metalloxid, das durch eine geeignete Heizung auf eine Arbeitstemperatur gebracht wird.

Große Verbreitung haben Zinndioxid-Sensorelemente gefunden, die auf eine Temperatur von ca. 250 Grad C bis 450 Grad C aufgeheizt werden. Dem Zinndioxid wird in der Regel eine katalytisch wirkende Substanz beigemischt. Bewährt hat sich z.B. Platin, Palladium, Rhodium.

Sensorelemente dieser Art reagieren mit einer Widerstandsverminderung bei der Anwesenheit von Gasen, die oxidierbar sind. Dabei gibt das Zinndioxid Sauerstoff ab. Wird der Sensor wieder normaler Luft ausgesetzt, reagiert die Oberfläche mit dem Luftsauerstoff wieder zu Zinndioxid. Der Vorgang ist also reversibel und unterliegt keinem Verschleiß.

Es wird beobachtet, daß mitunter eine grobe Differenz zwischen erwartetem Sensorleitwert und Konzentration oxidierbarer Gase, z.B. Kohlenmonoxid, auftritt. Nähere Untersuchungen haben jetzt gezeigt, daß bei gleichzeitiger Anwesenheit reduzierbarer Gase diese die Reaktion der oxidierbaren Gase mit der Sensoroberfläche stark beeinflussen. Im Extremfall wird trotz grosser Konzentration keine oder eine nur geringe elektrische Reaktion des Sensors erfolgen. Beispielhaft sei auf die Reaktion bei gleichzeitigem Vorhandensein von Kohlenmonoxid (CO) und Stickstoffoxid (NOx) hingewiesen. Ursache ist die direkte Reaktion der Gase miteinander in der Nähe der heißen Sensoroberfläche, wobei die Katalysatorsubstanz Einfluß nimmt.

Insbesondere ist bei dem vorstehend erwähnten beheizten Zinndioxid-Sensor nachteilig, daß er gegenüber Dieselabgasen wenig sensibel ist. Dies ergibt sich aus folgendem:

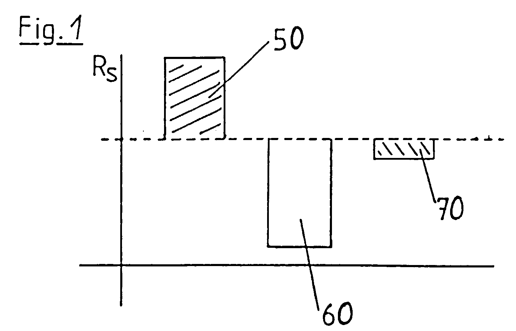

Zinndioxid-Sensoren reagieren mit einer Widerstandsverminderung immer dann, wenn eine oxidierbare, gasförmige Substanz anwesend ist. Zinndioxid-Sensoren reagieren also z.B. auf Kohlenmonoxid, Wasserstoff oder Benzindämpfe entsprechend der Darstellung des Balkens 60 in Figur 1. In den Abgasen von Benzinmotoren befinden sich diese Bstandteile, so daß es zu dieser deutlichen Reaktion des Zinndioxid-Sensors kommt.

Wird dieser Zinndioxid-Sensor im Labor mit Stickoxiden (NOx) beaufschlagt, erhöht er seinen inneren Widerstand, wie durch den Balken 50 in Figur 1 gezeigt.

Im Abgas eines Diesel-Motors, insbesondere wenn dieser unter Last betrieben wird, befinden sich neben den dem Balken 60 in Figur 1 entsprechenden Substanzen auch die dem Balken 50 entsprechenden Substanzen. Als Resultat wird beobachtet, daß der Zinndioxid-Sensor deutlich weniger reagiert, als man es aufgrund der meßtechnisch nachgewiesenen Gasanteile vermuten kann. Die Reaktion des Zinndioxid-Sensors entspricht dem Balken 70 in Figur 1.

Dies ist für den Betrieb des Sensors sehr störend, da beim Einsatz eines solchen Gas-Sensors in der Praxis häufig sowohl oxidierbare als auch reduzierbare Gase auftreten.

Aus der DE-C-32 13 286 ist eine Gas-Sensor-Anordnung zur Detektion von Gasen in der Luft bekannt, bei der bereits mehrere Sensorelemente an einem Träger vorgesehen sind, wobei es sich hierbei um unterschiedliche Sensorelemente, die unterschiedliche Stoffe erfassen sollen, handelt. Der Betrieb der Gas-Sensor-Anordnung erfolgt bei einer Temperatur, die für sämtliche verwendeten Sensorelemente gleichmäßig ist und so ausgewählt ist, daß alle Sensorelemente bei dieser Temperatur mit befriedigenden Ergebnissen funktionieren. Das heißt, bei dieser bekannten Gas-Sensor-Anordnung wird eine Temperatur ausgewählt, die für sämtliche vorgesehenen Sensorelemente akzeptabel ist.

Der Erfindung liegt die Aufgabe zugrunde, die gattungsgemäße Gas-Sensor-Anordnung derart weiterzubilden, daß sichergestellt ist, daß die Sensorelemente jeweils in dem für sie optimalen Bereich arbeiten können.

Diese Aufgabe wird erfindungsgemäß dadurch gelöst, daß das erste Sensorelement und das zweite Sensorelement durch eine SiO2-Trennfuge thermisch voneinander getrennt auf dem ihnen gemeinsamen Träger angeordnet sind. Durch diese thermische Trennung der beiden Sensorelemente wird sichergestellt, daß die beiden Sensorelemente der erfindungsgemäßen Gas-Sensor-Anordnung jeweils in dem für sie optimalen Temperaturbereich arbeiten können.

Gemäß Patentanspruch 2 macht sich die Erfindung die Beobachtung zunutze, daß Phthalocyanine zur Detektion insbesondere von reduzierbaren Gasen (z.B. NOx) geeignet sind. Aufgrund der relativ geringen Arbeitstemperatur dieser Sensorelemente ist die Querempfindlichkeit gegenüber oxidierbaren Gasen gering. Es wird daher vorgeschlagen, die Signale sowohl eines Metalloxid-Sensorelements, z.B. auf Zinndioxidbasis, als auch eines Phthalocyanin-Sensorelements gleichzeitig auszuwerten.

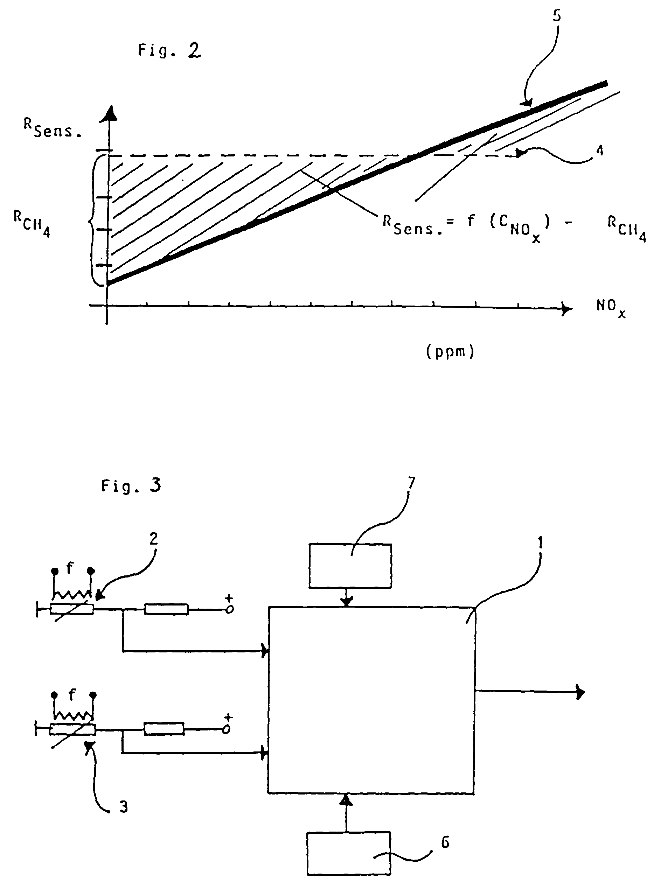

Figur 2 zeigt den prinzipiellen Zusammenhang, wobei der innere Sensorwiderstand 5 bei Anwesenheit einer konstanten Konzentration von Methan (CH4) und einer unterschiedlichen Konzentration von Stickstoffoxid (NO2) gezeigt wird. Als 100%-Widerstandswert 4 wird der Sensorwiderstand bei Normalluft bezeichnet.

Figur 3 zeigt eine Prinzipdarstellung eines Ausführungsbeispiels der Erfindung. Eine Auswerteeinheit 1 liest die Sensorsignale ein, die durch Reihenschaltung der Sensorelemente mit einem Außenwiderstand entstehen. Dabei ist 2 ein Zinndioxid-Sensor, der oxidierbare Gase erkennt, und 3 ein Phthalocyanin-Sensor, der auf reduzierbare Gase reagiert.

In der Auswerteeinheit, die sich vorteilhaft programmgesteuert eines Mikroprozessors bedient, werden die beiden Sensorsignale rechnerisch miteinander verknüpft.

Dabei wird - angelehnt an Figur 2 - das Sensorsignal des Zinndioxid-Sensors 2 rechnerisch (analog oder digital) von den Einflüssen oxidierender Gase befreit, die gleichzeitig am Phthalocyanin-Sensor 3 Signale erzeugen.

Wenn die Signalauswertung gleichzeitig auch mit Informationen über Temperatur und Luftfeuchte erfolgt, können die Quereinflüsse von Temperatur und Luftfeuchte ebenfalls ausgeglichen werden. In Figur 3 ist daher ein Feuchte-Sensor 7 und ein Temperatur-Sensor 6 dargestellt. Da der jeweilige Einfluß auf den jeweiligen Sensor bekannt ist, kann er z.B. in einer Korrekturtabelle in der Auswerteeinheit 1 abgelegt und berücksichtigt werden.

Bei einer bevorzugten Lösung wird Polysilizium als Träger benutzt, wobei das Polysilizium durch Anlegen einer elektrischen Spannung direkt als Heizelement genutzt werden kann. Vorteilhaft sichert eine solche Anordnung reproduzierbare thermische Verhältnisse für beide Sensortypen und steigert dadurch die Meßsicherheit.

Vorteilhaft verhindert die Erfindung, daß zu erkennende oxidierbare Gase durch gleichzeitige Anwesenheit reduzierbarer Gase maskiert werden und somit nicht oder verfälscht detektiert werden.

## Patentansprüche

1. Gas-Sensor-Anordnung zur Detektion von Gasen in der Luft, mit einem ersten Sensorelement (2), welches als Metalloxid-Sensorelement ausgebildet ist, hauptsächlich empfindlich gegenüber oxidierbaren Gasen ist und ein dementsprechendes Meßsignal erzeugt, einem zweiten Sensorelement (3), welches hauptsächlich empfindlich gegenüber reduzierbaren Gasen ist und ein dementsprechendes Meßsignal erzeugt, einer elektrischen Steuer- und Auswerteeinheit (10), in der die vom ersten Sensorelement (2) und vom zweiten Sensorelement (3) erzeugten Meßsignale ausgewertet werden, und einem Träger, der beheizbar ist und auf dem sowohl das erste Sensorelement (2) als auch das zweite Sensorelement (3) angeordnet sind, dadurch gekennzeichnet, daß das erste Sensorelement (2) und das zweite Sensorelement (3) durch eine SiO2-Trennfuge thermisch voneinander getrennt auf dem ihnen gemeinsamen Träger angeordnet sind.

2. Gas-Sensor-Anordnung nach Anspruch 1, die ein oder mehrere Metalloxid-Sensorelemente (2) und zusätzlich ein oder mehrere Phthalocyanin-Sensorelemente (3) enthält, wobei die Signale aller Sensorelemente (2, 3) in der Auswerteeinheit (10) so bewertet werden, daß die gegenseitige Beeinflussung gleichzeitig vorhandener reduzierbarer und oxidierbarer Gase aufgehoben wird, indem in der Auswerteeinheit (1) die Signale des bzw. der Metalloxid-Sensorelemente (2) mit den Signalen des bzw. der Phthalocyanin-Sensorelemente (3) entsprechend einer in der Auswerteeinheit (1) abgespeicherten Funktion verknüpft werden.

3. Gas-Sensor-Anordnung nach Anspruch 1 oder 2, bei der zusätzlich die Lufttemperatur und/oder die Luftfeuchte durch geeignete Sensorelemente (6, 7) ermittelt wird bzw. werden und zusätzlich der Auswerteeinheit (1) zugeführt wird bzw. werden, wobei der spezifische Einfluß dieser Parameter ebenfalls als Korrekturfunktion in der Auswerteeinheit (1) vorhanden ist und die Signale dieser Sensorelemente (6, 7) funktionsgerecht als Korrekturgrößen eingesetzt werden.

4. Gas-Sensor-Anordnung nach einem der Ansprüche 1 - 3, bei der als Trägermaterial für die Sensorelemente (2, 3) Polysilizium dient, auf das die Sensorelemente (2, 3) im Dünnfilmverfahren aufgebracht sind.

5. Gas-Sensor-Anordnung nach einem der Ansprüche 1 - 3, bei der als Trägermaterial für die Sensorelemente (2, 3) Aluminiumoxid dient, auf das die Sensorelemente (2, 3) und eine benötigte Heizung im Dickfilmverfahren aufgebracht sind.

6. Gas-Sensor-Anordnung nach einem der Ansprüche 1 - 5, bei der die Metalloxid-Sensorelemente (2) als Zinndioxid-Sensorelemente ausgebildet sind.

## Claims

1. Gas sensor arrangement for detecting gases in the air, having a first sensor element (2) which is in the form of a metal oxide sensor element, is principally sensitive to oxidisable gases and generates a corresponding measuring signal, a second sensor element (3) which is principally sensitive to reducible gases and generates a corresponding measuring signal, an electrical control and evaluation unit (10) in which the measuring signals generated by the first sensor element (2) and by the second sensor element (3) are evaluated, and a carrier which can be heated and on which both the first sensor element (2) and the second sensor element (3) are arranged, characterised in that the first sensor element (2) and the second sensor element (3) are arranged on the carrier, which is common to both of them, in such a manner that they are separated from one another thermally by an SiO₂ separating line.

2. Gas sensor arrangement according to Claim 1, which contains one or more metal oxide sensor elements (2) and, additionally, one or more phthalocyanine sensor elements (3), the signals of all the sensor elements (2, 3) being evaluated in the evaluation unit (10) in such a manner that the influence of simultaneously present reducible and oxidisable gases on one another is cancelled out by linking the signals of the metal oxide sensor element(s) (2) to the signals of the phthalocyanine sensor element(s) (3) in the evaluation unit (1) in accordance with a function stored in the evaluation unit (1).

3. Gas sensor arrangement according to Claim 1 or 2, in which, additionally, the air temperature and/or the air humidity is (are) determined by suitable sensor elements (6, 7) and additionally conveyed to the evaluation unit (1), wherein the specific influence of those parameters is also present as a correction function in the evaluation unit (1), and the signals of the sensor elements (6, 7) are used as correction values in accordance with the function.

4. Gas sensor arrangement according to any one of Claims 1 to 3, in which the carrier material used for the sensor elements (2, 3) is polysilicon to which the sensor elements (2, 3) are applied by the thin film process.

5. Gas sensor arrangement according to any one of Claims 1 to 3, in which the carrier material used for the sensor elements (2, 3) is aluminium oxide to which the sensor elements (2, 3) and the necessary heating are applied by the thick film process.

6. Gas sensor arrangement according to any one of Claims 1 to 5, in which the metal oxide sensor elements (2) are in the form of tin dioxide sensor elements.

## Revendications

1. Système détecteur de gaz pour la détection de gaz dans l'air, comportant un premier détecteur élémentaire (2) qui est réalisé sous la forme d'un détecteur à oxyde métallique, est essentiellement sensible aux gaz oxydables et délivre un signal de mesure correspondant, un second détecteur élémentaire (3) qui est essentiellement sensible aux gaz réductibles et délivre un signal de mesure correspondant, une unité (1) électrique de commande et de traitement, dans laquelle les signaux de mesure produits par les premier (2) et second (3) détecteurs élémentaires sont traités, et un support qui peut être chauffé et sur lequel sont disposés le premier détecteur élémentaire (2) et le second détecteur élémentaire (3), caractérisé par le fait que le premier détecteur élémentaire (2) et le second détecteur élémentaire (3) sont séparés thermiquement l'un de l'autre, sur le support commun, par une barrière de SiO₂.

2. Système détecteur de gaz selon la revendication 1, comportant un ou plusieurs détecteurs élémentaires (2) à oxyde métallique et, en outre, un ou plusieurs détecteurs élémentaires (3) à phtalocyanine, les signaux de tous les détecteurs élémentaires (2,3) étant traités dans l'unité de traitement (1) de manière telle que l'influence réciproque des gaz réductibles et des gaz oxydables présents simultanément soit annulée, le traitement des signaux étant opéré par chaînage dans l'unité de traitement (1), des signaux du ou des détecteur(s) élémentaire(s) (2) à oxyde métallique et des signaux du ou des détecteur(s) élémentaire(s) (3) à phtalocyanine, conformément à une fonction mémorisée dans l'unité de traitement (1).

3. Système détecteur de gaz selon la revendication 1 ou la revendication 2 dans lequel, en outre, la température de l'air et/ou l'humidité de l'air est/sont mesurée(s) à l'aide de détecteurs élémentaires (6, 7) adaptés et est/sont transmise(s) à l'unité de traitement (1), l'influence spécifique de ces paramètres étant également présente sous forme de fonction de correction dans l'unité de traitement et les signaux des détecteurs élémentaires (6, 7) étant utilisés conformément à la fonction, en tant que grandeurs de correction.

4. Système détecteur de gaz selon l'une des revendications 1 à 3, dans lequel on utilise comme matériau de support pour les détecteurs élémentaires (2, 3), du polysilicium sur lequel les détecteurs élémentaires (2, 3) sont appliqués par un procédé de dépôt en couches minces.

5. Système détecteur de gaz selon l'une des revendications 1 à 3, dans lequel on utilise comme matériau de support pour les détecteurs élémentaires (2, 3), de l'oxyde d'aluminium sur lequel les détecteurs élémentaires (2, 3) et un moyen de chauffage nécessaire sont appliqués par un procédé de dépôt en couches épaisses.

6. Système détecteur de gaz selon l'une des revendications 1 à 5, dans lequel les détecteurs élémentaires (2) à oxyde métallique sont des détecteurs élémentaires à oxyde stannique.
